# EUROPEAN PATENT APPLICATION

(11) **EP 3 235 926 A1**
(43) Date of publication of application: **25.10.2017**
(21) Application number: 17166865.0
(22) Date of filing: 18.04.2017
(51) Int. Cl.: C23C 26/00, A61L 15/00, D06M 11/36

(54) **SONOCHEMICAL COATING METHOD**

(30) Priority: 18.04.2016 US 201615132042
(71) Applicant: Bar-Ilan University, 5290002 Ramat Gan (IL)
(72) Inventor: Aharon, Gedanken, Givataim 5346521 (IL); Ilana, Perelshtein, Rishon Lezion 7570046 (IL); Nina, Perkas, Petach Tikva 4963211 (IL)
(74) Representative: RGTH

(57) **Abstract**

In order to provide an efficient, fast, versatile and reliable method for preparing antibacterial, antimicrobial or antifungal surfaces a sonochemical coating method, in particular for preparing antibacterial, antimicrobial or antifungal surfaces, is proposed, comprising the steps of
- providing a mixture comprising water,
- immersing a substrate in the mixture,
- Embedding metal oxide nanoparticles contained/or formed in the mixture in a surface of the substrate and/or depositing metal oxide nanoparticles contained and/or formed in the mixture on a surface of the substrate by irradiating the mixture with ultrasonic waves.

## Description

The present invention relates to a sonochemical coating method, in particular for preparing antibacterial, antimicrobial or antifungal surfaces. Furthermore, the present invention relates to a sonochemical method for preparing a substrate with embedded nanoparticles and to a substrate coated or embedded with nanoparticles.

### Prior art

Antimicrobial, antibacterial or antifungal fabrics are widely used for production of outdoor clothes, underwear, bed-linen and bandages. Antimicrobial resistance is very important in textile materials, having effects amongst others on comfort for the wearer. The deposition of metal oxides known to possess antimicrobial activity, in particular zinc oxide (ZnO), magnesium oxide (MgO) or copper oxide (CuO), can significantly extend the applications of substrates, such as textile fabrics, and prolong the period of their use.

Catheter-associated urinary tract infections (CAUTI) are a major class of nosocomial infections. Catheterization can facilitate colonization of the urethra by internal microbiota and environmental bacteria and promote biofilm development, often resulting in bacteriuria. Many bacterial species that colonize catheters can form biofilms and are associated with serious complications. Although prevention strategies following the recommended guidelines, such as assessment of the catheter need, practice of aseptic insertion technique, and shorter catheterization durations, have succeeded in significantly reducing CAUTI rates, target rates have not yet been met.

Zinc oxide (ZnO) has been recognized as a mild antimicrobial agent, non toxic wound healing agent, and sunscreen agent. Because it reflects both UVA and UVB rays, zinc oxide can be used in ointments, creams and lotions to protect against sunburn and other damage to the skin caused by ultraviolet lights [Godfrey H.R. Alternative Therapy Health Medicine, 7 (2001) 49]. At the same time ZnO is an inorganic oxide and stable against temperatures encountered in normal textile use, contributing to its long functional lifetime without color change or oxidation. The antibacterial properties of MgO and CuO nanoparticles were also demonstrated [Controllable preparation of Nano-MgO and investigation of its bactericidal properties. Huang L., Li D.Q, Lin Y. J., Wei M., Evans D.G., Duan X. L. Inorganic Biochemistry, 99 (2005) 986, and Antibacterial Vermiculite Nano-Material. Li B., Yu S., Hwang J. Y., Shi S. Journal of Minerals & Materials Characterization & Engineering, 1 (2002) 61].

An antimicrobial formulation containing ZnO powder, binding agent, and dispersing agent was used to protect cotton and cotton-polyester fabrics ["Microbial Detection, Surface Morphology, and Thermal Stability of Cotton and Cotton/Polyester Fabrics Treated with Antimicrobial Formulations by a Radiation Method". Zohby M. H., Kareem H. A., El-Naggar A. M., Hassan, M. S., J. Appl. Polym. Sci. 89 (2003) 2604]. This formulation was applied to fabrics under high energy radiation of Co-60 γ or electron beam irradiation and then subjected for fixation by thermal treatment. In spite of good antimicrobial activity, the disadvantages of this method are the use of additional binding and dispersing agent, and requirements of high energy radiation and an additional stage of thermal curing. It was also reported that ZnO-soluble starch nanocomposite was impregnated onto cotton fabrics to impart antibacterial and UV-protection functions [Functional finishing of cotton fabrics using zinc oxide - soluble starch nanocomposites. Vigneshwaran N., Kumar S., Kathe A. A., Varadarajan P., Prasad V., Nanotechnology 17 (2006) 5087]. However, in this work the special stabilizing agent, namely acrylic binder, is used which should undergo the additional stage of polymerization at 140°C.

Improved methods for preparing antibacterial, antimicrobial or antifungal surfaces are still a long felt need.

### Summary of the invention: problem, solution, advantages

It is the object of the present invention to provide an efficient, fast, versatile and reliable method for preparing antibacterial, antimicrobial or antifungal substrates and surfaces thereof. Furthermore, it is an object of the present invention to provide a substrate coated or embedded with metal oxide nanoparticles.

To solve the problem a sonochemical coating method, in particular for preparing antibacterial, antimicrobial or antifungal surfaces, is proposed, comprising the steps of
- providing a mixture comprising water,
- immersing a substrate in the mixture,
- embedding metal oxide nanoparticles contained and/or formed in the mixture in a surface of the substrate and/or depositing metal oxide nanoparticles contained and/or formed in the mixture on a surface of the substrate by irradiating the mixture with ultrasonic waves.

The sonochemical coating method is conducted in the presence of a substrate. According to the predominant explanation the embedding and/or the depositing of the nanoparticles is a result of acoustic bubbles being formed in the mixture by the ultrasonic waves in the sonochemical coating method. The acoustic bubbles tend to collapse preferentially near the surface, in particular near a solid surface, of a substrate. The after-effects of the collapse of the acoustic bubbles are microjets and shockwaves directed towards the surfaces. Nanoparticles formed and/or contained in the mixture are thrown at the surface by the microjets. As a result the metal oxide nanoparticles are strongly anchored to or embedded in the surface of the substrate without the use of any binding agent. The nanoparticles may already be present in the mixture when the substrate is immersed therein. However, it may also be the case that the nanoparticles are formed or produced in the mixture from a metal oxide precursor using energy from the collapse of the acoustic bubbles after the substrate is immersed in the mixture. The collapse of the acoustic bubbles provides the energy for forming the metal oxide nanoparticles. For this to happen, metal oxide precursors, for example a metal acetate, may be present in the mixture.

With regard to the metal oxide nanoparticles, the expressions "formed" or "produced" may be used interchangeably.

The use of the proposed sonochemical coating method shortens preparation time, is inexpensive and does not require complicated equipment since it preferably may be carried out in a chemical solution by controlling concentration of the reagents and the reaction time.

Surprisingly, the proposed sonochemical coating method allows coating of virtually any suitable surface of any suitable substrate from any suitable material. Thus, any suitable substrate can be reliably coated with the proposed sonochemical coating method.

Advantageously, the metal oxide nanoparticles may be formed or produced "in situ" in the mixture from metal oxide precursors and by the collapse of acoustic bubbles during conducting the sonochemical coating method, or the metal oxide nanoparticles may be produced prior to conducting the sonochemical coating method and simply be added to the mixture. These possibilities provide a great versatility and flexibility to the sonochemical coating method and lower the costs.

Another one of the many advantages of the sonochemical coating method is that a homogeneous dispersion of the nanoparticles on the surface of the substrate is achieved in one step. A further advantage of using ultrasonic waves over other methods is that the shockwaves from the collapse of the acoustic bubbles effectively blast the metal oxide nanoparticles onto the surface of the substrate at such speed that it causes local melting of the substrate, guaranteeing a firm embedding of the nanoparticles within the surface. Surfaces sonochemically coated or impregnated with metal oxides, in particular with ZnO nanoparticles, display outstanding antimicrobial activity in the case of both gram positive and gram negative bacteria.

For examples, tests with textiles coated with ZnO using the proposed sonochemical coating method have shown that the textiles demonstrate high stability. The amount of, for example, ZnO remaining in the textile after 50 or even after 65 washing cycles remains constant. The stability of nanoparticles on the fabric was measured after 50 or even after 65 washing cycles by both HRSEM measurements and probing the concentration of ZnO by ICP. The amount of remaining ZnO may refer to the amount of active or antibacterial active ZnO. Thus, though the amount of nanoparticles may decrease during the washing cycles, the amount of antibacterial active nanoparticles remains effectively constant.

A metal oxide nanoparticle is a nanoparticle which at least comprises a metal oxide or consists of a metal oxide. A metal oxide nanoparticle may also comprise additional elements, in particular additional metallic elements. A metal oxide comprises one or more metal atoms and one or more oxygen atoms, wherein one or more of the metal atoms is in association with one or more oxygen atoms. The metal oxide can include an oxygen to metal atomic ratio ranging from 4:1 to 1:1.

Instead of water any other suitable liquid may be used, in particular liquids in which acoustic bubbles can be formed by ultrasonic waves. Thus, throughout this description the term "water" may be replaced by the term "liquid". Thus, a more general embodiment of the inventive sonochemical coating method, in particular for preparing antibacterial, antimicrobial or antifungal surfaces, comprises the steps of
- providing a mixture comprising a liquid,
- immersing a substrate in the mixture,
- embedding metal oxide nanoparticles contained and/or formed in the mixture in a surface of the substrate and/or depositing metal oxide nanoparticles contained and/or formed in the mixture on a surface of the substrate by irradiating the mixture with ultrasonic waves.

The liquid may be an alcohol, for example but not limited to ethanol, iso-propyl alcohol or ethylene glycol or the liquid may be an organic solvent, for example but not limited to dodecan, dimethylformamide (DMF) or heptan.

However most preferably the liquid is water.

In the following the invention is described with water as the liquid, however instead of water any other suitable liquid may be employed instead of water.

Preferably, the metal oxide nanoparticle may comprise or consist of, without limitation, oxides of magnesium, titanium, aluminum, zirconium, calcium, scandium, vanadium, chromium, manganese, ferrite, cobalt, nickel, copper and zinc.

Particularly preferably the metal oxide nanoparticle comprises or consists of copper oxide (CuO), zinc oxide (ZnO) or magnesium oxide (MgO).

A nanoparticle, in particular a metal oxide nanoparticle, is a particle featuring a size of at least one dimension that ranges from about 1 nm to 1000 nm. Particularly the term nanoparticle refers to particles of size ranging from about 10 nanometers to about 10 micrometers.

The term nanoparticle may also refer to particles having an average size between 1 nm to 500 nm, preferably between 1 nm to 200 nm, further preferably between 1 nm to 50 nm and most preferably below 35 nm. Very particularly preferably the average nanoparticle size ranges from 3 to 5 nm.

Preferably, the step of providing the mixture comprises the step of adding metal oxide nanoparticles to the water.

Thus, advantageously the metal oxide nanoparticles are not produced in the mixture during conducting the sonochemical coating method but are added to the mixture before the mixture is irradiated with ultrasonic waves. In other words, pre-produced or commercially available metal oxide nanoparticles are introduced in the mixture. The pre-produced metal oxide nanoparticles may be, however, produced with a separate sonochemical procedure. By irradiating the mixture comprising or containing the metal oxide nanoparticles these nanoparticles are embedded in the surface of the substrate or deposited on the surface of the substrate. This way of conducting the sonochemical coating method may also be referred to as the "throwing stones method".

Further preferably, the step of irradiating the mixture is carried out by means of an ultrasonic transducer. The ultrasonic transducer may be configured rectangular, cylindrical or in the form of a horn, in particular of a titanium ultrasonic horn (Ti-horn). The transducer may be in any form operated at an appropriate energy.

Still further preferably the ultrasound frequency of the ultrasonic waves ranges from 10 kHz to 100 kHz, more preferably from 15 kHz to 80 kHz, particular preferably from 20 kHz to 50 kHz. Most preferably the step of irradiating the mixture can be carried out using ultrasonic waves at a frequency of approximately 20 kHz, in particular at a frequency of 20 kHz.

It may be preferred that the power of the ultrasonic wave radiation is between 100 W and 10 kW, further preferably between 500 W and 5 kW, more preferably between 1 kW and 2kW. Furthermore, the step of irradiating the mixture can be carried out using ultrasonic waves at a power of approximately 1.5 kW. Most preferably the power of the ultrasonic wave radiation is 750 W or 1.5 kW. The power of the ultrasonic wave radiation may reach up to or more than 4 kW, in particular for scale-up transducers.

Preferably the intensity of the ultrasonic wave radiation is between 10 W/cm² and 500 W/cm², preferably between 20 W/cm² and 100 W/cm², still more preferably between 40 W/cm² and 50 W/cm². Most preferably the intensity of the ultrasonic wave radiation is 45 W/cm².

The mixture may be irradiated with ultrasonic waves for a duration between 1 min and 10 h, preferably between 10 min and 5 h, more preferably between 30 min and 2h. Most preferably the mixture is irradiated with ultrasonic waves for 30 min or 1h. Accordingly, the step of irradiating the mixture can be carried out for 1 hour. The mixture also may be irradiated for at least 30 min, preferably for at least 1h.

Preferably the mixture consists solely of water and metal oxide nanoparticles.

This is particularly advantageous when the sonochemical coating method is conducted using the "throwing stones" technique. Advantageously, by adding only pre-produced and/or commercially available nanoparticles to the water to form a mixture, a very simple mixture is used allowing for a simple, fast and efficient coating process.

It is furthermore preferred that the metal oxide nanoparticles are doped metal oxide nanoparticles.

A doped metal oxide nanoparticle is a nanoparticle composite comprising a metal oxide and ions of a metallic element included in a crystal lattice of the metal oxide. The metallic element, also called the Dopant, may be, for example and without limitation, magnesium, copper, zinc, titanium, aluminum, scandium, vanadium, chromium, manganese, ferrous, cobalt, nickel, zirconium, hafnium, calcium, yttrium, or gallium.

The inclusion of such metallic elements in the crystal lattice of the metal oxide is also referred to herein and in the art as "doping", with the metallic element referred to as "Dopant". For example, if the metal oxide is CuO with Zn as the metallic element included in a crystal lattice of the metal oxide, Zn is the Dopant.

The doped metal oxide nanoparticle formed upon inclusion of ions of a different metallic element is also referred to herein and in the art as "crystal lattice-doped metal oxide" or simply as "doped metal oxide".

Preferably the metallic element is copper, zinc, and/or magnesium. More preferably the metallic element is different from the metal in the metal oxide.

Still further preferably the metal oxide is copper oxide and the metallic element is zinc, and/or the metal oxide is magnesium oxide and the metallic element is zinc, and/or the metal oxide is copper oxide and the metallic element is magnesium.

Preferably an atomic ratio of the metal of the metal oxide and the ions of the metallic element, i.e. the Dopant, in the doped metal oxide nanoparticle ranges from 20:1 to 4:1, more preferably from 10:1 to 4:1, particularly preferably the atomic ratio of the metal of the metal oxide and the ions of the metallic element is 8:1.

Furthermore preferably, the atomic ratio of the metal of the metal oxide and the metallic element, i.e. the Dopant, in the doped metal oxide nanoparticle or in the crystal-lattice doped metal oxide ranges from 10:1 to 4:1 or from 10:1 to 5:1, and can be, for example, 10:1, 9:1, 8:1, 7:1, 6:1, 5:1 or 4:1, including any value there between.

For example, in case of CuO, i.e. the metal oxide, doped with Zn the atomic ratio of the metal of the metal oxide in the doped metal oxide nanoparticle and the metallic element, i.e the atomic ratio Cu:Zn, may be 8:1.

Advantageously, with the use of pre-produced or commercially available doped metal oxide nanoparticles the antibacterial, antimicrobial or antifungal properties are improved compared to the pristine undoped metal oxide nanoparticles.

The doped metal oxide nanoparticles may, however, also be produced "in situ" in the mixture by the collapse of acoustic bubbles during conducting of the sonochemical coating method.

Preferably, the step of providing the mixture comprises the steps of providing a solution comprising water and a soluble metal oxide precursor, for example a corresponding metal acetate, wherein the metal oxide nanoparticles are formed or produced by the irradiation of the mixture with ultrasonic waves, wherein preferably the solution further comprises a soluble metal Dopant precursor, wherein in particular preferably the doped metal oxide nanoparticles are produced by the irradiation of the mixture with ultrasonic waves.

Thus, a solution comprising water and a soluble metal oxide precursor and preferably further comprising a metal Dopant precursor is provided. By irradiating the mixture comprising the solution with ultrasonic waves, metal oxide nanoparticles are produced or, if a metal Dopant precursor is also contained in the solution, doped metal oxide nanoparticles are produced sonochemically. The irradiation of the mixture comprising the solution may be conducted before the substrate is immersed into the mixture or after the substrate is immersed into the mixture. Most preferably, the metal oxide nanoparticles or the doped metal oxide nanoparticles are produced by irradiating the mixture with ultrasonic waves in the step of embedding the metal oxide nanoparticles in the surface of the substrate and/or depositing the metal oxide nanoparticles on the surface of the substrate.

Preferably the molar ratio of the soluble metal oxide precursor and the soluble metal Dopant precursor, in particular of a soluble metal oxide precursor salt and a soluble metal Dopant precursor salt, ranges from 50:1 to 1:50, or from 40:1 to 1:40, or from 30:1 to 1:30, or from 20:1 to 1:20, or from 12:1 to 1:12.

Furthermore preferably, the molar ratio of metal oxide precursor and the metal Dopant precursor, in particular of a soluble metal oxide precursor salt and a soluble metal Dopant precursor salt, is 12:1, 11:1, 10:1, 9:1, 8:1, 7:1, 6:1, 5:1, 4:1, 3:1, 2:1, 1:1, 1:12, 1:11, 1:10, 1:9, 1:8, 1:7, 1:6, 1:5, 1:4, 1:3, or 1:2, including any value therebetween.

Particularly preferably the molar ratio of metal oxide precursor and the metal Dopant precursor, in particular of a soluble metal oxide precursor salt and a soluble metal Dopant precursor salt, is 3:1.

For example, if the metal oxide precursor is a soluble salt of Copper and the metal Dopant precursor is a soluble salt of Zink, the ratio Cu:Zn is between 12:1 and 1:2, preferably between 5:1 and 1:1, particularly preferably the ratio Cu:Zn is 3:1.

Advantageously, with the sonochemical coating method all the principle requirements on an antimicrobial surface coated with nanoparticles can be achieved: a small particle size, a regular shape and a homogeneous distribution of (doped) metal oxide nanoparticles on the surface of the substrate.

It may furthermore be preferred that the solution consists solely of water and the soluble metal oxide precursor, or that the solution preferably consists solely of water and the soluble metal oxide precursor and the soluble metal Dopant precursor.

By limiting the solution to only comprise water, a soluble metal oxide precursor and preferably a soluble metal Dopant precursor, a very simple and pure coating process is provided, which results in a particularly homogeneous coated surface.

Preferably the solution and/or the mixture may contain no additional solvent, in particular the solution and/or the mixture may contain no ethanol. Particularly preferably the solution and/or the mixture is not and/or does not comprise a water ethanol solution and/or no water ethanol solution is present and or used in the sonochemical coating method.

A sonochemical coating method, in which the solution and/or the mixture contains no additional solvent, in particular no ethanol, is called a "pure water" sonochemical coating method. Metal oxide nanoparticles may also be present in the "pure water" sonochemical coating method, in particular the metal oxide nanoparticles which are produced or formed during conducting the method.

Surprisingly, it has been found by the applicant that the sonochemical coating method can also be conducted in pure water mixed only with the soluble metal oxide precursor and/or the soluble metal Dopant precursor and/or metal oxide nanoparticles.

However, it is also possible that the solution contains an additional substance or compound, for example a solvent.

Still further preferably the mixtures solely consist of the solution.

Thus, no additional substances need to be added to the mixture and the mixture may be identical to the solution, which only comprises water, nanoparticles and/or a metal oxide precursor and/or a metal Dopant precursor.

Preferably, the sonochemical coating method is configured so that the coated substrate contains 0.005 to 10.0 wt. %, preferably 0.1 to 10.0 wt. %, more preferably 3.0 to 7.0 wt. %, further preferably 4.0 to 6.0 wt. %, of metal oxide, in particular of metal oxide nanoparticles. For medical devices, for example for catheters, the medical device may contain 0.05 wt. % of metal oxide.

Preferably, the sonochemical coating method is configured so that in case of a textile as the substrate the textile contains 0.1 to 10.0 wt. %, more preferably 3.0 to 7.0 wt. %, further preferably 4.0 to 6.0 wt. %, of metal oxide, in particular of metal oxide nanoparticles. In particular preferably the textile may contain 0.7 wt. % of metal oxide.

It may be preferred that pre-produced or commercially available metal oxide nanoparticles or doped metal oxide nanoparticles maybe added to the solution or the mixture at any time during conducting the sonochemical coating process.

Preferably, the method comprises the step of adjusting the pH of the mixture, wherein the pH is adjusted to be further preferably basic.

Expediently, the pH is adjusted to at least 7, more preferably the pH is adjusted to between 7 to 12, further preferably between 8 and 10, most preferably the pH is adjusted to 8. Expediently, the basic pH may be approximately 8.

When the sonochemical coating method is conducted according to the throwing stones method, i.e. when pre-produced and/or commercially available metal oxide nanoparticles are added to the mixture, the pH does not need to be adjusted and may take any possible value. However, in the throwing stones method it is preferred that the pH value is between 6 and 8, in particular 7.

When the metal oxide nanoparticles are synthesized from metal oxide precursors by irradiating the mixture with ultrasonic waves, the pH of the mixture may be adjusted to a value between 8 to 10 in particular to 8. In a further preferred embodiment, the pH of the mixture is between 8.5 to 9.

The pH can be adjusted by adding a basic compound to the mixture, in particular aqueous ammonia.

By adding aqueous ammonia to the mixture in the in situ sonochemical coating method, the production of metal oxide nanoparticles, in particular from the metal oxide precursors and/or from the metal Dopant precursors, is advantageously efficient.

However, known methods other than addition of ammonia may be used to adjust the pH of the mixture.

Preferably the mixture consists solely of water and metal oxide nanoparticles and the basic compound, and/or the mixture consists solely of the solution and the basic compound.

It has to be noted that while the solution may consist solely of water and the soluble metal oxide precursor and/or the soluble metal Dopant precursor the mixture may consist of the solution and of the basic compound. Thus, the sonochemical coating method may be conducted as a pure water sonochemical coating method with the pH adjusted by adding a basic compound, for example ammonia.

In a further preferred embodiment, the mixture and/or the solution may comprise a water miscible solvent, in particular ethanol.

By providing a water-ethanol solution and/or a water-ethanol mixture, the rate of production of the metal oxide nanoparticles or of the doped metal oxide nanoparticles is increased resulting in a faster, more efficient and more homogeneous coating of the surface of the immersed substrate.

It may be preferred that the water - ethanol solution, in particular the water-ethanol ratio, is in a ratio of between 1:1 and 1:12, more preferably between 1:7 and 1:10, most preferably of approximately 1:9.

Furthermore, solvents other than ethanol may be used instead of or in addition to ethanol in preparing the solution. A number of different solutions may be used, depending on the application and particulars of the other steps of the process.

Preferably the sonochemical coating method further comprises the step of purging the mixture, in particular to remove traces of gases, in particular of air and/or CO₂, from the mixture.
The purging of the mixture may be performed using a noble gas, in particular argon.

The purging may proceed for at least 10 min, preferably for at least 30 min, most preferably for at least 1 h.

Furthermore preferably, the step of purging is carried out with argon for 1 hour.

Furthermore, it can be preferred that the step of embedding the metal oxide nanoparticles in the surface of the substrate and/or disposing the metal oxide nanoparticles on the surface of the substrate is done under a constant flow of a noble gas, in particular argon. Thus, the step of irradiating the mixture may be carried out under a flow of argon.

Still more preferably, the method may comprise the step of pre-treating the surface of the substrate with a chemical treatment and/or a physical treatment and/or a plasma treatment, wherein particularly preferably the plasma treatment uses an oxygen plasma and/or an oxygen argon plasma.

Preferably the chemical treatment comprises etching of the substrate, in particular of the surface of the substrate, with an acid.

In particular for substrates made of silicon or of a polymer or of any hydrophobic surface, such as medical devices, a plasma treatment, expediently a plasma pretreatment, may be conducted in order to activate the surface of said substrate.

By activating the surface of the substrate, a deeper, longer lasting and permanent embedding or coating of metal oxide nanoparticle in or on the surface of the substrate is achieved.

Preferably, the substrate may comprise or consists of an organic material and/or an inorganic material. The substrate may be or comprise glass, paper, a ceramic, a polymer, metal, leather, cotton, silk or any other suitable material.

Surprisingly, it has been found by the applicant that any suitable surface can be coated using the sonochemical coating method, in particular using the pure water sonochemical coating method or the throwing stones method. The substrate made of or comprising the suitable material may be coated in a mixture which only consists of a solution, which consists solely of water and a metal oxide precursor and preferably a metal Dopant precursor, (pure water sonochemical coating method) and, if applicable, a basic compound. With the sonochemical coating method it has become possible to coat any suitable surface in a "pure water environment".

Furthermore, the sonochemical coating method may be configured to coat porcelain surfaces, ceramic surfaces, silicon or organosilicon surfaces, metallic surfaces (e.g., stainless steel), MICA, polymeric surfaces such as, for example, plastic surfaces, rubbery surfaces, paper, wood, fabric in a woven, knitted or non-woven form, mineral (rock or glass), surfaces, wool, silk, cotton, hemp, leather, fur, feather, skin, hide, pelt or pelage surfaces, plastic surfaces and surfaces comprising or made of polymers such as but not limited to polypropylene (PP), polycarbonate (PC), high-density polyethylene (HDPE) polyester (PE), unplasticized polyvinyl chloride (PVC), and fluoropolymers including but not limited to polytetrafluoroethylene (PTFE, Teflon^{®}). The substrate or the surface of the substrate can comprise or be made of any of the foregoing substances, or any mixture thereof.

In addition, it is preferred that the substrate is a medical device, a textile, a prosthesis, a microelectronic device, a formulation, in particular a cream or a lotion.

The substrates may include, but are not limited to, medical devices, pharmaceutical, cosmetic or cosmeceutic products, fabrics, bandages, microelectronic devices, microelectromechanic devices, photovoltaic devices, microfluidic devices, articles having a corrosivable surface, agricultural devices, packages, sealing articles, fuel containers and construction elements.

Non-limiting examples of medical devices which can beneficially be coated with metal oxide nanoparticle using the sonochemical coating method include catheters, tubing, endotracheal tubing, vaginal devices such as tampons, prosthetic devices, medical or cosmetic implants, artificial joints, artificial valves, needles, intravenous access devices, cannula, stents, biliary stents, cardiovascular stents, cardiac surgery devices, nephrostomy tubes, vascular grafts, infusion pumps, adhesive patches, sutures, fabrics, meshes, polymeric surgical tools or instruments, intubation devices, orthopedic surgery devices, pacemakers, endoscope components, dental surgery devices, veterinary surgery devices, bone scaffolds, hemodialysis tubing or equipment, blood exchanging and transfusion devices, implantable prostheses, bandages, ophthalmic devices, wound dressings, breast implants, heart valves, replacement joints, catheters, catheter access ports, dialysis tubing, gastric bands, shunts, screw plates, artificial spinal disc replacements, internal implantable defibrillators, cardiac resynchronization therapy devices, implantable cardiac monitors, mitral valve ring repair devices, left ventricular assist devices (LVADs), artificial hearts, implantable infusion pumps, implantable insulin pumps, stents, implantable neurostimulators, maxillofacial implants, dental implants, artificial teeth and the like.

Preferably the medical device is an implantable medical device, including medical devices that are implanted transiently or permanently. Examples include an indwelling catheter or an intubation device such as a tracheal tube. Non-limiting examples of indwelling catheters include urinary catheters, central venous catheters, biliary vascular catheters, pulmonary artery catheters, peripheral venous catheters, arterial lines, central venous catheters, peritoneal catheters, epidural catheters and central nervous system catheters.

The medical device may be made of any of the suitable polymeric materials described hereinabove (e.g., at least a portion of the medical device comprises a polymeric material).

The substrate may be a formulation, in particular in form of a paste, a cream, a lotion, a foam, a gel, an emulsion, an ointment, a soap, a pladget, a swab, a suppository, a dressing, a solution, a mousse, a pad, a wipe, a patch, a milk, an oil, a suspension, an aerosol, or a spray.

Still further preferably the metal oxide precursor is a water soluble salt of the metal of the metal oxide, in particular an acetate, a nitrate, or a chloride of said metal.

Furthermore, it is preferred that the metal Dopant precursor is a water soluble salt of the metallic element, preferably an acetate, a nitrate, or a chloride of the metallic element, in particular of the Dopant.

Furthermore, the water soluble salt maybe a bromine salt, an iodine salt, a sulfate salt or a hydroxide salt or any other water soluble salt. The water soluble salt may be utilized in form of a hydrate, for example a monohydrate, a dihydrate, a trihydrate or a tetrahydrate.

For example, in case of zinc as the metal in the metal oxide and/or as the Dopant the water soluble zinc salt maybe without limitation a zinc acetate, zinc nitrate, zinc chloride, zinc bromide, zinc sulfate, zinc chlorate, zinc chloride or zinc hydroxide, zinc acetate dihydrate, zinc acetate monohydrate etc.

Preferably, the metal oxide precursor is added in a concentration between 0.002 to 0.5 M, preferably between 0.01 to 0.02 M, most preferably to 0.015 M to the solution, preferably to water, wherein M denotes the molarity in units of mol per liter.

Preferably the sonochemical coating method comprises the step of adding M(Ac)₂ to a water-ethanol solution, where M stands for metals Zn, Mg, Cu; and Ac stands for acetate ion, where M(Ac)₂ is added in a concentration of between 0.002 and 0.02 M.

It is further preferable that M is selected from a group consisting of the metals Zn, Mg, Cu.

Preferably the molar ratio of the water soluble salt of the metal oxide precursor, for example of the copper acetate monohydrate, and the water soluble salt of the Dopant precursor, for example of the zinc acetate dihydrate, ranges from 12:1 to 1:12.

More preferably, the molar ratio of the water soluble salt of the metal oxide precursor, for example of the copper acetate monohydrate, and the water soluble salt of the Dopant precursor, for example of the zinc acetate dihydrate, is 12:1, 11:1, 10:1, 9:1, 8:1, 7:1, 6:1, 5:1, 4:1, 3:1, 2:1, 1:1, 1:12, 1:11, 1:10, 1:9, 1:8, 1:7, 1:6, 1:5, 1:4, 1:3, or 1:2, including any value therebetween.

Particularly preferably the molar ratio of the water soluble salt of the metal oxide precursor, for example of the copper acetate monohydrate, and the water soluble salt of the Dopant precursor, for example of the zinc acetate dihydrate, is 3:1.

Expediently, the molar ratio of the metal of the water soluble salt of the metal oxide precursor and the metal of the water soluble salt of the Dopant precursor is the ratio in the solution.

Expediently, the temperature of the mixture during conducting the sonochemical method, in particular during the step of irradiating the mixture with ultrasonic waves is at least 20°C, preferably at least 30°C, more preferably at least 50°C, particular preferably approximately or at least 70°C.

It can be preferred that the step of irradiating the mixture is carried out at approximately 30°C.

For the sonochemical coating method conducted as a "throwing stones method", the coating reaction occurs preferably between 25°C to 75°C.

In the in situ sonochemical coating method, in which the metal oxide nanoparticles are produced by irradiating a mixture comprising a solution of water and a metal oxide precursor, a temperature of 50°C is expediently required before the addition of a basic compound, in particular of ammonia, to the mixture. The reaction temperature than may reach approximately 70°C.

Furthermore, the temperature maybe at least 90°C.

In a particular preferred embodiment, the temperature of the mixture during the irradiation is between 60°C and 80°C.

Another solution to the object of the present invention is the provision of a sonochemical method for preparing a substrate with embedded nanoparticles, wherein the method comprises the steps of
- providing a mixture, wherein the mixture comprises a solution comprising water and a metal oxide precursor, preferably further comprising a metal Dopant precursor,
- producing metal oxide nanoparticles, preferably doped metal oxide nanoparticles, by irradiating the mixture with ultrasonic waves,
- isolating the nanoparticles, preferably by drying the irradiated mixture,
- embedding the produced nanoparticles in the substrate by mixing the nanoparticles with the substrate.

The metal oxide nanoparticles are preferably produced by irradiating the mixture, wherein the mixture comprises a solution, wherein the solution solely consists of water and a metal oxide precursor. The mixture may additionally contain a basic compound, such as aqueous ammonia. Advantageously, the production of nanoparticles does not use a water miscible solvent such as ethanol.

Preferably the substrate is a formulation, in particular a medical formulation, or an antimicrobial and/or antifungal cream or lotion or a gel. Particularly preferable the substrate is a paste, a cream, a lotion, a foam, a gel, an emulsion, an ointment, a soap, a pladget, a swab, a suppository, a dressing, a solution, a mousse, a pad, a wipe, a patch, a milk, an oil, a suspension, an aerosol, or a spray.

Thus, an antifungal, antimicrobial, medical formulation can be provided which contains the metal oxide nanoparticles or doped metal oxide nanoparticles produced with the sonochemical method.

The sonochemical method for preparing a substrate with embedded nanoparticles may furthermore comprise any of the steps of any of the methods described above, possibly without the step of immersing a substrate in the mixture, and if applicable without the step of embedding metal oxide nanoparticles contained in the mixture in a surface of the substrate and/or depositing metal oxide nanoparticles contained in the mixture on a surface of the substrate by irradiating the mixture with ultrasonic waves.

The production of metal oxide nanoparticles by irradiating the mixture with ultrasonic waves may, however, simultaneously proceed with a second substrate being immersed into the mixture and, preferably additionally or alternatively pre-produced or commercially available metal oxide nanoparticles being added to the mixture.

A further solution to the object of the present invention is the provision of a substrate obtainable and/or coated or embedded with nanoparticles using the sonochemical method as described above.

### Short description of the figures

The invention is explained with reference to the figures.
- Fig. 1: presents a flow diagram of a first embodiment of a sonochemical coating method,
- Fig. 2: presents a flow diagram of a second embodiment of a sonochemical coating method,
- Fig. 3: presents an HRSEM image of a textile coated with copper oxide nanoparticles,
- Fig. 4: presents an SEM image of a silicon catheter coated with copper oxide nanoparticles,
- Fig. 5: presents a flow diagram of a third embodiment of a sonochemical coating method, and
- Fig. 6: presents a flow diagram of a special embodiment of the second sonochemical coating method.

### Detailed explanation of the figures

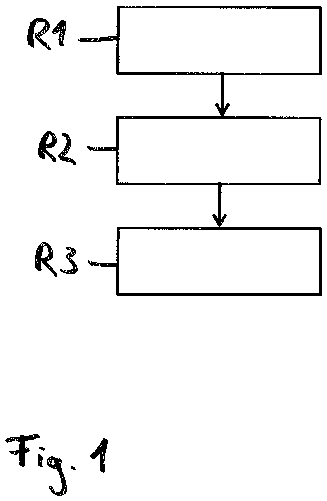

Fig. 1 shows a flow diagram of an embodiment of the sonochemical coating method.

In a first step R1 a mixture comprising water is provided. The mixture comprising water in the depicted embodiment consists solely of a solution which consists of water and a soluble metal oxide precursor, such as copper acetate monohydrate. In particular, no ethanol is contained in the mixture or the solution. The method shown in fig. 1 is a variant of the "pure water" sonochemical coating method.

In a second step R2 a substrate, for example a medical device such as a catheter, is immersed in the mixture.

In a third step R3 the mixture with the substrate being immersed therein is irradiated with ultrasonic waves. Due to the ultrasonic waves acoustic bubbles are created in the mixture, which afterwards collapse. The collapsing acoustic bubbles provide the energy for forming metal oxide nanoparticles, in particular copper oxide nanoparticles, from the metal oxide precursor in the mixture.

Since the acoustic bubbles collapse preferentially near the surface of the substrate, the formed metal oxide nanoparticles are deposited on or embedded in the surface of the substrate.

In the sonochemical coating method shown in fig. 1 only water and a metal oxide precursor are contained in the mixture. Thus no ethanol or basic compound to adjust the pH of the mixture are added to the mixture. However, in a variant of the pure water sonochemical coating method, a basic compound such as ammonia is added to the mixture to adjust the pH, for example to a value between 8 and 8.5. Still, in this variant no ethanol is added to the mixture.

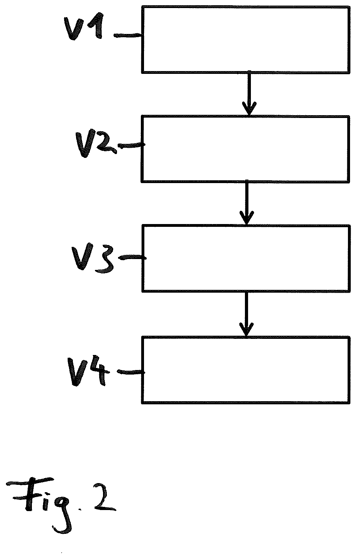

Fig. 2 shows a flow diagram of the "throwing stones" sonochemical coating method.

In a first step V1 for providing a mixture, water is provided. In a second step V2 pre-produced metal oxide nanoparticles, for example copper oxide nanoparticles, are added to the water. Thus, a mixture containing solely water and pre-produced metal oxide nanoparticles is provided. The pre-produced metal oxide nanoparticles may be preferably CuO nanoparticles or doped CuO nanoparticles, in particular CuO nanoparticles doped with Zn.

In a third step V3 a substrate, for example a medical device such as a catheter, is immersed into the mixture. In a fourth step V4 the mixture consisting only of water and the metal oxide nanoparticles is irradiated with ultrasonic waves. Due to the ultrasonic waves acoustic bubbles form and collapse preferentially close to the surface of the immersed substrate. The collapse of the acoustic bubbles results in microjets and shockwaves which are directed towards the surface of the substrate. The nanoparticles contained in the mixture are thrown at the surface by the microjets and, thus, the metal oxide nanoparticles, in particular the copper oxide nanoparticles, are embedded in or deposited on the surface of the substrate.

Similar to the "pure water" coating method of fig. 1, the "throwing stones" sonochemical coating method of figure 2 is realized without the addition of ethanol or of a basic compound to adjust the pH of the mixture.

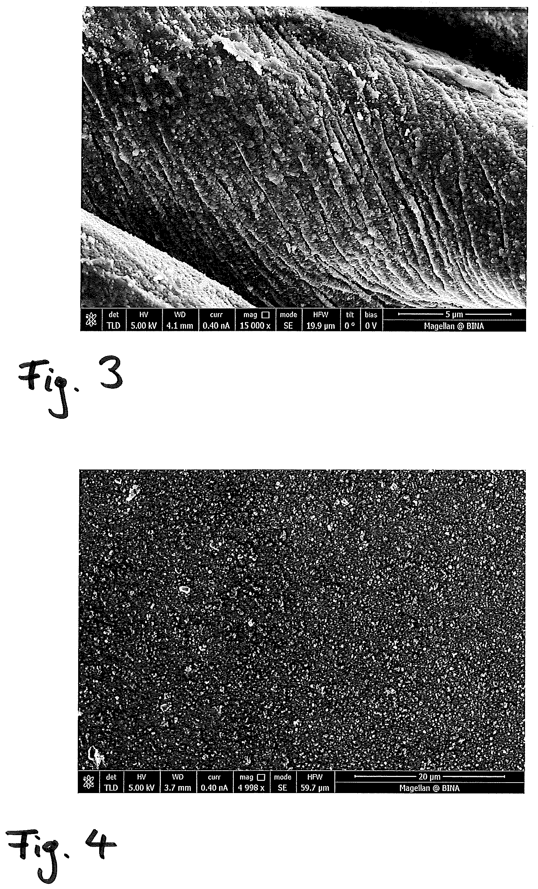

Fig. 3 shows an HRSEM image of a textile coated with copper oxide nanoparticles with the variant of the "pure water" sonochemical coating method according to fig. 1, wherein copper oxide nanoparticles are synthesized in a mixture consisting of a solution and a basic compound, wherein the solution consists only of water and a metal oxide precursor. The copper oxide nanoparticles formed in the mixture are homogeneously distributed on the surface of the textile.

Fig. 4 is an SEM image of a silicon catheter coated with copper oxide nanoparticles using the "pure water" sonochemical coating method. The catheter was immersed into the mixture consisting of water and the metal oxide precursor, namely copper acetate monohydrate. The mixture has been irradiated with ultrasonic waves. The copper oxide nanoparticles produce by the collapse of acoustic bubbles from the ultrasonic waves are thrown at the surface of the catheter and embedded in or disposed on the surface of the catheter. As shown in fig. 4, the nanoparticles are coated homogeneously on the surface of the silicon catheter.

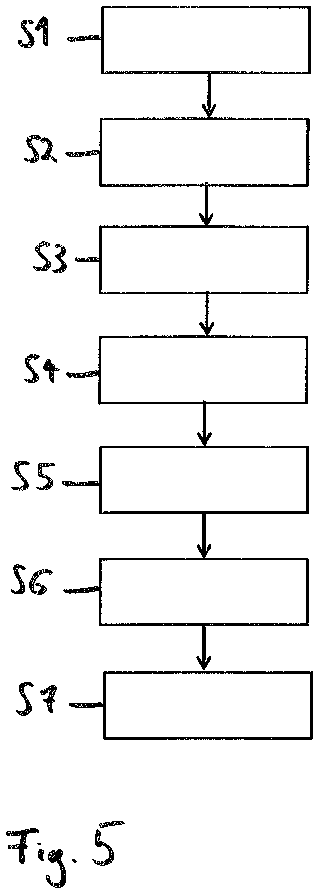

Fig. 5 shows a flow diagram of another embodiment of the sonochemical coating method. In a first step S1, for providing a solution a metal oxide precursor M(Ac)₂, for example a zinc acetate, is added to water, where M indicates the metal, in this case zinc, and Ac stands for the acetate ion. No further substances are added to the solution. The so prepared solution is used as the mixture and thus forms the mixture for the sonochemical coating method. In a second step S2, a substrate is immersed into said mixture. Subsequently in step S3, the pH of the mixture is adjusted to basic pH of approximately 8 by adding aqueous ammonia. Afterwards, the mixture is purged to remove traces of CO₂ and/or air with argon for about one hour in step S4.

In step S5 the mixture is irradiated with a high intensity ultrasonic power in the form of ultrasonic waves. Metal oxide nanoparticles are formed sonochemically using the ultrasonic energy. Collapsing acoustic bubbles formed by the ultrasonic waves result in microjets which embed the produced nanoparticles in or dispose the nanoparticles on the surface of the substrate. Afterwards, in step S6 the surface is washed with water to remove traces of ammonia. In step S7 the surface is further washed with ethanol, and dried in air.

Hence, the sonochemical coating method shown in fig. 5 comprises the steps
- S1: adding M(Ac)₂ to water, forming a mixture;
- S2: immersing said surface in said mixture;
- S3: adjusting the pH of said mixture to basic pH by means of addition of aqueous ammonia;
- S4: purging said mixture to remove traces of CO₂/air;
- S5: irradiating said mixture with a high intensity ultrasonic power;
- S6: washing said surface with water to remove traces of ammonia;
- S7: further washing said surface with ethanol, and drying in air.

Thus, a surface - metal oxide composite is produced containing and homogeneously impregnated with metal oxide nanoparticles, without use of electromagnetic radiation. Step S4 is optional and good results are obtained without it.

The water based reaction may be used for simultaneous production of metal oxide nanoparticles and coating of various substrates with the antibacterial metal oxide nanoparticles. In a one-step reaction the water solution of the corresponding metal (Zn⁺² or Cu⁺²) undergoes a hydrolysis process in a basic environment (pH ∼ 8).

The method without steps S2, S6 and S7 may be used to produce nanoparticles without applying them to a surface, while steps S2 to S7 may be used with an additional step of adding nanoparticles, in particular metal oxide nanoparticles, to the mixture to coat surfaces with nanoparticles without simultaneously producing nanoparticles. The surface may be any type of material, including ceramics, polymers, metals, glass, textile and/or paper and applications include medical devices etc. By avoiding the use of ethanol in the mixture, potential toxicity of nanoparticles due to the use of ethanol during the synthesis process and its aggregation during interaction with salts can be avoided. Fires and other accidents during bulk scale nanoparticle synthesis may also be prevented and the process becomes more economical (water is typically cheaper than ethanol and the resulting process is less complex) and more environmentally friendly (without the need to dispose of ethanol waste).

Nanoparticles synthesized in water may have different shape and size characteristics than those synthesized in a water-ethanol solution, however the functionality of coatings of the nanoparticles is unaffected by these variations in size and shape. The water based method may be used for applying Mg-based nanoparticles (e.g. commercially obtained nanoparticles) to a surface of a substrate.

When commercially obtained nanoparticles, or nanoparticles produced by means other than sonochemically, are introduced in the mixture, in particular in the sonication mixture, the ultrasound, in particular the ultrasonic waves, can still be used for "throwing stones" at the surface, for example at a fabric surface or at a medical device, and good antibacterial properties (when antibacterial nanoparticles, such as metal oxide nanoparticles, are used) are obtained.

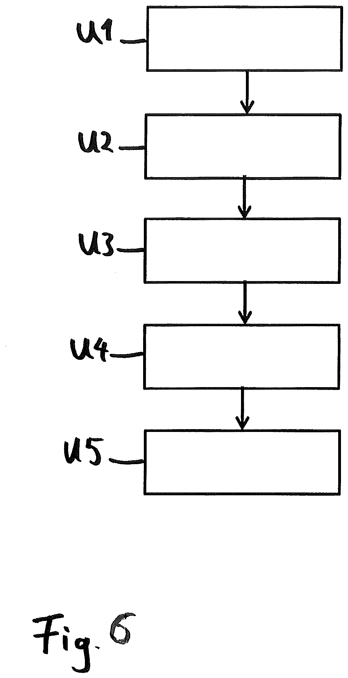

Fig. 6 shows another embodiment of the sonochemical coating method. The sonochemical coating method of Fig. 6 comprises the steps of:
- U1: A sample (such as a cotton square of about 100 cm²) is placed in a 0.002 - 0.02 M solution of M(Ac)₂ (where M stands for metals Zn, Mg, Cu; and Ac stands for acetate ion) in water.
- U2: The pH is adjusted to 8 with an aqueous solution of ammonia.
- U3: The reaction mixture is then purged with argon for 1 hour in order to remove traces of CO₂/air.
- U4: The solution is irradiated for 1 hour with a high intensity ultrasonic horn (Ti-horn, 20 kHz, 1.5 kW at 70 % efficiency) under a flow of argon at 30°C.
- U5: The textile is washed thoroughly with water to remove traces of ammonia, then further washed with ethanol and dried in air.

Metal solutions as above may be prepared using metal nitrates or other salts, as will be obvious to one skilled in the art. Furthermore, the sonochemical coating method shown in Fig. 6 can be conducted in a wide range of values for the molarity M, the pH, the purging time with argon or other gases, the irradiation time with ultrasonic waves as well as the power, efficiency and frequency of the ultrasonic horn. In particular, the values of these parameters may take the values as herein disclosed.

Either of the methods shown in Figs. 5 or 6 may be modified to replace the first steps S1 or U1 (with regard to the feature of providing a solution of M(Ac)₂), with a step of adding commercial/pre-produced metal oxide nanoparticles to the water, or the pre-produced metal oxide nanoparticles may be added as an additional step.

As will also be obvious to one skilled in the art, the coating process can be accomplished without producing nanoparticles 'in house', by adding nanoparticles obtained by some other means to the water and ultrasonically treating as above in steps U2 to U5. The yield (amount of nanoparticles on the sample, in particular the textile) in this case could be lower but enough to get antibacterial properties.

## Claims

1. Sonochemical coating method, in particular for preparing antibacterial, antimicrobial or antifungal surfaces, comprising the steps of
- providing a mixture comprising water,
- immersing a substrate in the mixture,
- Embedding metal oxide nanoparticles contained and/or formed in the mixture in a surface of the substrate and/or depositing metal oxide nanoparticles contained and/or formed in the mixture on a surface of the substrate by irradiating the mixture with ultrasonic waves.

2. Sonochemical coating method according to claim 1, wherein the step of providing the mixture comprises the step of adding metal oxide nanoparticles to the water.

3. Sonochemical coating method according to claim 1 or 2, wherein the mixture consists solely of water and metal oxide nanoparticles.

4. Sonochemical coating method according to any one of the preceding claims, wherein the metal oxide nanoparticle comprises or consists of copper oxide (CuO), zinc oxide (ZnO) or magnesium oxide (MgO) or mixtures thereof, and/or wherein the metal oxide nanoparticles are doped metal oxide nanoparticles.

5. Sonochemical coating method according to any one of the preceding claims, wherein the step of providing the mixture comprises the steps of providing a solution comprising water and a soluble metal oxide precursor, wherein the metal oxide nanoparticles are produced by the irradiation of the mixture with ultrasonic waves, wherein the solution preferably further comprises a soluble metal Dopant precursor, wherein in particular preferably the doped metal oxide nanoparticles are produced by the irradiation of the mixture with ultrasonic waves.

6. Sonochemical coating method according to claim 5, wherein the solution consists solely of water and the soluble metal oxide precursor, or wherein the solution preferably consists solely of water and the soluble metal oxide precursor and the metal Dopant precursor.

7. Sonochemical coating method according to any one of the preceding claims, wherein the solution and/or the mixture contains no additional solvent, in particular no ethanol.

8. Sonochemical coating method according to any one of claims 5 to 7, wherein the mixture solely consists of the solution.

9. Sonochemical coating method according to any one of the preceding claims, wherein the method comprises the step of adjusting the pH of the mixture, wherein the pH is adjusted to be preferably basic, and/or wherein a basic compound is added, preferably aqueous ammonia, wherein further preferably the pH is adjusted by adding the basic compound, wherein the mixture particularly preferably consists solely of water and metal oxide nanoparticles and the basic compound, and/or wherein the mixture consists solely of the solution and the basic compound, and/or wherein the mixture and/or the solution comprises a water miscible solvent, in particular ethanol.

10. Sonochemical coating method according to any one of the preceding claims, wherein the method further comprises the step of purging the mixture, in particular to remove traces of gases, in particular of air and/or CO₂, from the mixture, and/or wherein the method comprises the step of pre-treating the surface of the substrate with a chemical treatment, preferably etching of the substrate, in particular of the surface of the substrate, with an acid, and/or a physical treatment and/or a plasma treatment, wherein the plasma treatment particularly preferably uses an oxygen plasma and/or an oxygen-argon plasma.

11. Sonochemical coating method according to any one of the preceding claims, wherein the substrate comprises or consists of an organic material and/or inorganic material, in particular glass, paper, a ceramic, a polymer, metal, leather, cotton, silk, and/or wherein the substrate is a medical device, a textile, a prosthesis, a microelectronic device, a formulation, in particular a cream or a lotion, a pharmaceutical, cosmetic or cosmeceutic product, a fabric, a bandage, a microelectromechanic device, a photovoltaic devices, a microfluidic device, an article having a corrosivable surface, an agricultural device, a package, a sealing article, a fuel container or a construction element.

12. Sonochemical coating method according to according to any one of claims 5 to 11, wherein the metal oxide precursor is a water-soluble salt of the metal of the metal oxide, in particular an acetate, a nitrate, or a chloride of said metal, and/or wherein the metal Dopant precursor is an acetate, a nitrate, a chloride of the metallic element, in particular of the Dopant.

13. Sonochemical method for preparing a substrate with embedded nanoparticles, wherein the method comprises the steps of
- providing a mixture, wherein the mixture comprises a solution comprising water and a metal oxide precursor, preferably further comprising a metal Dopant precursor,
- producing metal oxide nanoparticles, preferably doped metal oxide nanoparticles, by irradiating the mixture with ultrasonic waves,
- isolating the nanoparticles, preferably by drying the irradiated mixture,
- embedding the produced nanoparticles in the substrate by mixing the nanoparticles with the substrate.

14. Sonochemical method according to claim 13, wherein the substrate is a formulation, in particular a medical formulation, preferably an antimicrobial and/or antifungal cream or lotion or gel, and/or wherein the method comprises any of the steps of any one of the claims 1 to 12.

15. Substrate coated or embedded with nanoparticles obtainable and/or produced with anyone of the sonochemical methods of claims 1 to 14.
